# DEMANDE DE BREVET EUROPEEN

(11) **EP 3 243 433 A1**
(43) Date de publication de la demande: **15.11.2017**
(21) Numéro de dépôt: 17169392.2
(22) Date de dépôt: 04.05.2017
(51) Int. Cl.: A61B 5/103, A61B 5/107

(54) **DISPOSITIF NUMERIQUE DE MESURE PHYSIOLOGIQUE**

(30) Priorité: 11.05.2016 FR 1654187
(71) Demandeur: SARL SP, 38500 Voiron (FR)
(72) Inventeur: BAUDERE, David, 38500 VOIRON (FR); VALLON, Dimitri, 38500 VOIRON (FR)
(74) Mandataire: Cabinet Laurent & Charras

(57) **Abrégé**

L'invention concerne un dispositif numérique de mesure physiologique (10) comportant :
- une surface translucide (11) comportant une face supérieure (12) apte à recevoir un élément physiologique à mesurer et une face inférieure (13), opposée à la face supérieure;
- une source lumineuse (15) en regard de la tranche de ladite surface translucide de sorte qu'une lumière soit injectée dans la matière de ladite surface translucide pour révéler les zones d'appui dudit élément physiologique ; et
- un appareil de capture d'images (16) configuré pour capturer une image desdites zones d'appui dudit élément physiologique et positionné pour observer ladite face inférieure de ladite surface translucide,
- ladite face supérieure étant recouverte d'un complexe (18) comportant :
- un film polymérique (19) glacé, venant au contact de ladite face supérieure, et
- une couche de support (20) opaque, fixée avec ledit film polymérique, configurée pour recevoir ledit élément physiologique.

## Description

### DOMAINE TECHNIQUE

La présente invention concerne un dispositif de mesure physiologique, c'est-à-dire un dispositif permettant de mesurer une grandeur physique géométrique d'un organisme vivant tel que la taille ou la forme d'une partie du corps d'un individu ou d'un animal.

La présente invention concerne plus particulièrement un dispositif numérique de mesure physiologique, c'est-à-dire un dispositif dont la mesure est effectuée par des moyens numériques tels qu'une caméra ou un appareil photo.

L'invention trouve une application particulièrement avantageuse pour évaluer l'empreinte de la voute plantaire d'un être humain tout en mesurant de manière précise les grandeurs caractéristique de son pied.

### ART ANTERIEUR

Pour visualiser une empreinte de la voute plantaire d'un être humain, il est connu d'utiliser un podoscope. Cet appareil est composé d'une vitre épaisse configurée pour supporter le poids d'un patient, d'un dispositif d'éclairage tangentiel et d'un miroir placé au fond de l'appareil. La lumière du dispositif d'éclairage est injectée dans l'âme de la vitre épaisse réalisant ainsi un guide de l'onde lumineuse.

Pour utiliser le podoscope, un patient se tient debout sur la vitre et exerce par son poids des pressions sur la vitre avec certaines parties de son pied nu. Ce sont ces parties qui constituent les zones d'appui de la voûte plantaire. Ces zones d'appui créent un contraste avec les autres parties de la plante du pied qui ne viennent pas au contact direct de la vitre et ce contraste est mis en évidence par la réflexion de la lumière guidée dans l'âme de la vitre. Il est donc nécessaire que le pied du patient soit nu. Le miroir permet au podologue, qui pratique l'examen, d'observer ces zones d'appui de la voûte plantaire, en statique comme en dynamique, par réflexion de la lumière fixée sur la vitre. Le miroir est avantageusement incliné afin de permettre au podologue de se placer devant l'appareil.

Les zones d'appui de la voûte plantaire permettent de détecter la forme générale de la voute plantaire.

Le confort et le maintien du pied peut ainsi être amélioré grâce à des supports plantaires fabriqués selon les informations recueillies sur le pied du patient pendant l'examen.

Les études récentes ont montré qu'environ 70% de la population mondiale souffre de problèmes au niveau du pied. Il existe donc un besoin généralisé de mesure de la voute plantaire afin de guider les consommateurs vers l'utilisation de chaussures adaptées à leurs morphologies. Ainsi, il est recherché une méthode d'automatisation des mesures des caractéristiques physiologiques du pied d'un consommateur souhaitant acheter des chaussures ou des semelles adaptées à la marche ou à la pratique d'un sport en particulier, tel que des chaussons de ski.

Il existe des podoscopes numériques réalisés avec des dizaines de capteurs de pression et un logiciel informatique permettant de modéliser visualement l'empreinte plantaire et ses sollicitations en fonction de l'enfoncement de chaque capteur de pression sous le poids d'un utilisateur. Un tel podoscope numérique est décrit dans la demande de brevet américain N° US 2013/0174445. Cependant, le temps et le coût de réalisation de ces podoscopes numériques est prohibitif en raison du nombre de capteurs de pression requis pour obtenir un résultat satisfaisant.

La demande de brevet internationale N° WO 2008/006937 divulgue l'utilisation d'un podoscope classique intégrant un miroir sous la vitre, et grâce auquel le reflet de l'empreinte plantaire est capté par une caméra positionnée au-dessus de la surface vitrée sur laquelle le consommateur est installé. Un traitement numérique de la mesure effectuée par la caméra permet ainsi d'obtenir une mesure de l'empreinte plantaire. Cependant, ce podoscope numérique n'est pas adapté au déploiement à grand échelle car il est particulièrement sensible aux lumières environnantes pouvant se refléter sur la surface vitrée et le miroir associé.

Ainsi, l'utilisation de ce podoscope numérique dans un magasin nécessite l'utilisation des traitements numériques particulièrement longs et sensibles aux erreurs. Il n'est donc pas possible d'obtenir une visualisation des résultats en dynamique en raison des traitements numériques à effectuer.

En outre, ce podoscope numérique présente le même inconvénient que le podoscope classique, c'est-à-dire que l'utilisateur doit être pieds nus pour que les réflexions de la lumière soient efficaces. Il s'ensuit que ce podoscope numérique présente un problème d'hygiène lors d'utilisations successives par plusieurs utilisateurs dont les surfaces plantaires sont au contact de la même surface vitrée.

Le problème technique que propose de résoudre l'invention est de relever numériquement une mesure physiologique en limitant le bruit extérieur et en améliorant l'hygiène de la prise de mesure.

### EXPOSE DE L'INVENTION

La présente invention vise à résoudre ce problème technique en déplaçant l'appareil de capture d'images en dessous de la surface translucide et en recouvrant la surface translucide d'un complexe opaque de sorte à limiter le bruit extérieur et à améliorer l'hygiène de la prise de mesure.

A cet effet, l'invention concerne un dispositif numérique de mesure physiologique comportant :
- une surface translucide comportant une face supérieure apte à recevoir un élément physiologique à mesurer et une face inférieure, opposée à la face supérieure ;
- une source lumineuse en regard de la tranche de ladite surface translucide de sorte qu'une lumière soit injectée dans la matière de ladite surface translucide pour révéler les zones d'appui dudit élément physiologique ; et
- un appareil de capture d'images configuré pour capturer une image desdites zones d'appui dudit élément physiologique.

L'invention se caractérise en ce que ledit appareil de capture d'images est positionné pour observer ladite face inférieure de ladite surface translucide, ladite face supérieure étant recouverte d'un complexe comportant :
- un film polymérique glacé, venant au contact de ladite face supérieure, et
- une couche de support opaque, fixée avec ledit film polymérique, configurée pour recevoir ledit élément physiologique.

Pour réaliser l'invention, un grand nombre de matières ont été testées. Il ressort de ces essais que les films polymériques sont particulièrement adaptés pour révéler l'empreinte d'un élément physiologique, tel qu'un pied d'un individu. En effet, les films polymériques présentent l'avantage d'être compressibles est déformables afin de fixer la lumière guidée dans la surface translucide sans modifier de manière trop importante la forme de l'empreinte.

Cependant, certains films polymériques présentent un effet ventouse lorsqu'ils sont plaqués contre la surface translucide. Il s'ensuit qu'il est nécessaire de décoller ces films polymériques avant d'effectuer une nouvelle mesure. Au sens de l'invention, un film polymérique glacé est un film dont le traitement de surface est tel qu'il peut se décoller automatiquement de la surface translucide.

La couche de support opaque permet, quant à elle, de filtrer la lumière externe au dispositif. L'empreinte de l'élément physiologique est ainsi captée sur un fond uni et ne nécessite pas de traitements numériques. La rapidité de la mesure est également améliorée en raison d'un allégement des traitements numériques.

En outre, la couche de support permet de positionner l'élément physiologique en limitant les contacts entre l'élément physiologique et la surface translucide. Par exemple, dans le cas d'un podoscope numérique, la fixation de la lumière étant effectuée par la déformation du film polymérique, il n'est plus nécessaire d'effectuer la mesure avec les pieds nus. Les utilisateurs successifs peuvent donc conserver leurs chaussettes ce qui limite la propagation de bactéries et est un gain de temps dans l'enchainement des analyses. L'invention permet également de prévoir une couche de support lavable afin de limiter encore la propagation de bactéries. La couche de support peut éventuellement être remplacée pour permettre le lavage sans limiter l'utilisation du dispositif.

Selon un mode de réalisation, ledit complexe comporte également une couche compressible disposée entre ledit film polymérique et ladite couche de support.

Ce mode de réalisation permet d'améliorer la liaison entre ladite couche de support et ledit film polymérique. Par exemple, un collage peut être effectué entre ladite couche de support et ladite couche compressible et un collage peut être effectué entre ladite couche compressible et ladite couche polymérique alors que le collage entre ladite couche polymérique et ladite couche de support peut être difficile.

Au sens de l'invention, ladite couche compressible est configurée pour transmettre la contrainte subie sur sa face supérieure au film polymérique tout en limitant l'expansion de la zone de contrainte. En effet, si la couche est trop rigide, l'empreinte transmise au film polymérique sera déformée et la mesure sera erronée.

Selon un mode de réalisation, ladite couche de support correspond à un tricot réalisé à base de polyamide et d'élasthanne. La structure d'une étoffe de type tricot est particulièrement adaptée pour se déformer, et ainsi transmettre la forme de l'empreinte dudit élément physiologique en limitant sa déformation.

Selon un mode de réalisation, ladite couche de support est de couleur blanche. Ce mode de réalisation augmente le contraste de l'image capturée par l'appareil de capture d'images.

Selon un mode de réalisation, ledit film polymérique est réalisé à base de polyéthersulfone translucide, et dont l'épaisseur est comprise entre 10 et 50µm. Ce film polymérique est particulièrement adapté pour transmettre la forme de l'empreinte dudit élément physiologique tout en limitant l'effet ventouse contre ladite surface translucide.

Selon un mode de réalisation, ledit dispositif numérique comporte un second appareil de capture d'images positionné pour observer par-dessus ladite couche de support de sorte à mesurer une longueur et/ou une largueur dudit élément physiologique.

Ce mode de réalisation permet d'effectuer une double capture des caractéristiques physiologiques.

Dans le cas d'un podoscope numérique, le premier appareil de capture d'images permet de visualiser la forme de la voute plantaire alors que le second appareil de capture d'images permet d'estimer la pointure et la largeur du pied. Pour ce faire, le second appareil de capture est relié à une unité centrale qui effectue un traitement d'images simple. En effet, en connaissant la longueur et la largeur de la surface translucide, il est possible de déduire la pointure en fonction de la longueur du pied de l'utilisateur sur la surface translucide. Pour guider cette mesure, le pied peut être positionné en butée sur une cale.

Selon un mode de réalisation, ledit dispositif numérique comporte une seconde source lumineuse orientée vers ladite couche de support de sorte à limiter les ombres autour dudit élément physiologique pour les mesures effectuées par ledit second appareil de capture d'images. Ce mode de réalisation est particulièrement adapté à l'utilisation dans un magasin dans lequel les lumières sont nombreuses et créent des ombres parasites.

Selon un mode de réalisation, ladite couche de support comporte une surface supérieure grenelée de sorte à limiter les réflexions de la lumière sur ladite couche de support pour les mesures effectuées par ledit second appareil de capture d'images. Ce mode de réalisation limite également la perturbation de la mesure engendrée par les lumières externes.

Selon un mode de réalisation, ladite couche de support est de couleur verte. Ce mode de réalisation permet d'utiliser les algorithmes de filtrage classiques configurés pour extraire un corps sur un fond vert.

Selon un mode de réalisation, ladite couche de support est réalisée à base de polycarbonate. Ce mode de réalisation est particulièrement adapté pour protéger ledit film polymérique et pour garantir l'opacité du support.

Selon un mode de réalisation, ledit dispositif numérique est un podoscope numérique. Avantageusement, ledit dispositif numérique comporte un écran tactile comportant une interface de dialogue avec un utilisateur afin de le guider lors de la prise de mesures. Avantageusement, ledit dispositif numérique comporte une caméra apte à capturer et transmettre la position des jambes d'un utilisateur au cours du temps afin de contrôler que les jambes de l'utilisateur soient dans une position conforme à une prise de mesure.

### DESCRIPTION SOMMAIRE DES FIGURES

La manière de réaliser l'invention ainsi que les avantages qui en découlent, ressortiront bien du mode de réalisation qui suit, donné à titre indicatif mais non limitatif, à l'appui des figures annexées dans lesquelles les figures 1 à 2 représentent :
- figure 1 : une représentation schématique en perspective de face d'un podoscope numérique selon un mode de réalisation de l'invention ; et
- figure 2 : une vue en coupe de la partie basse du podoscope numérique de la figure 1.

### DESCRIPTION DETAILLEE DE L'INVENTION

La présente invention est décrite en référence à l'utilisation d'un podoscope numérique. Cependant, l'invention concerne, plus généralement, tous types de dispositifs numériques de mesure physiologique. Ainsi, la description peut être adaptée pour mesurer la forme d'une main d'un individu ou encore tout ou partie du corps d'un animal.

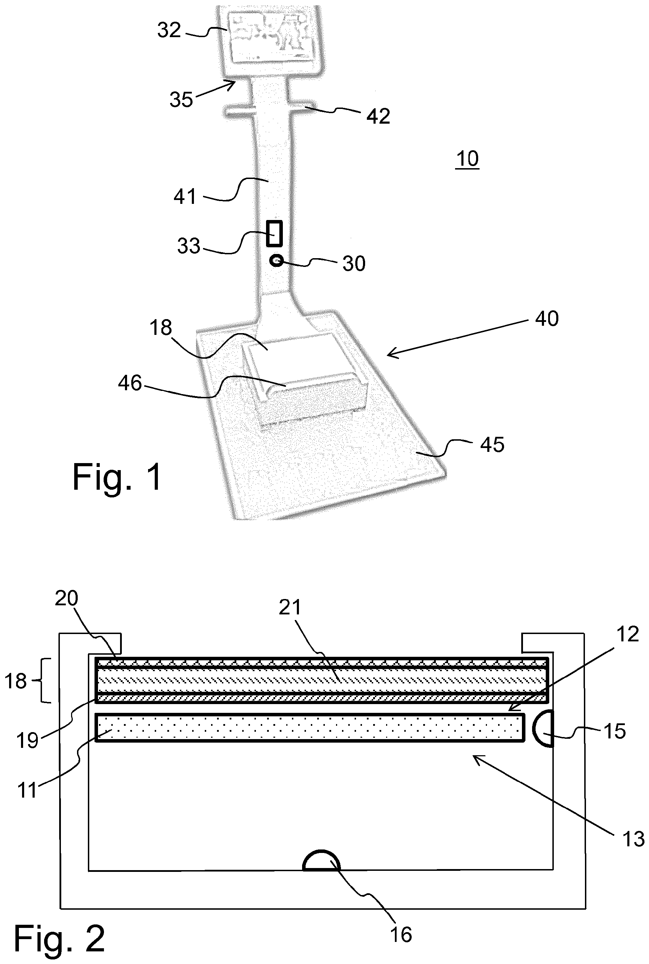

La figure 1 illustre un podoscope numérique **10** comportant une partie basse **40**, formant le pied du podoscope numérique **10**, au bord duquel s'élève un montant **41** configuré pour soutenir un écran tactile **32**. L'écran tactile **32** permet de guider un utilisateur pour mesurer les caractéristiques physiologiques de ses pieds. Lorsqu'un utilisateur touche l'écran tactile pour la première fois, la séquence de mesure commence. L'utilisateur est invité à se déchausser sur un banc positionné proche du podoscope numérique **10** et à évoluer en chaussettes sur un tapis **45** sur lequel le podoscope numérique **10** est disposé. L'utilisateur monte ensuite sur la partie basse **40** du podoscope numérique **10** en positionnant ses pieds avec le talon en butée contre une cale **46** s'étendant sur un bord de la partie basse **40** opposée au bord sur lequel le montant **41** est fixé. L'utilisateur est ainsi positionné face à l'écran tactile **32**.

Des poignées **42** sont montées de part et d'autre du montant **41** pour aider l'utilisateur à positionner correctement ses pieds. Le positionnement de l'utilisateur peut également être guidé par une caméra **35** disposée sous l'écran tactile **32**. Un flux vidéo de cette caméra **35** est alors transmis sur l'écran tactile afin de contrôler la position des jambes et des pieds de l'utilisateur. En effet, il est nécessaire que les pieds soient sensiblement droit pour mesurer leurs longueurs et il est nécessaire que les jambes soient sensiblement droite pour mesure la forme de la voûte plantaire.

Lorsque le positionnement est correct, les mesures sont effectuées. Pour ce faire, tel qu'illustré sur la figure 2, l'utilisateur est positionné sur un complexe **18** disposé sur une face supérieure **12** d'une surface translucide **11**. La surface translucide **11** est préférentiellement réalisée à base de verre ou de plexiglas®. Une source lumineuse **15**, par exemple un réseau de LED, est disposé en regard de la tranche de la surface translucide **11** de sorte à injecter une lumière dans la surface translucide **11** à la manière d'un podoscope classique.

Contrairement à un podoscope classique, les zones d'appui sont révélées par un film polymérique **19** glacé formant la couche inférieure du complexe **18**. Le film polymérique **19** est préférentiellement réalisé à base de polyethersulfone translucide, dont l'épaisseur est comprise entre 10 et 50 µm. Encore plus préférentiellement, l'épaisseur du film polymérique **19** est sensiblement égale à 20 µm. Ce film polymérique **19** est collé sur toute sa face supérieure avec une couche compressible **21**. Cette couche compressible **21** peut être réalisée avec plusieurs matières sans impacter le fonctionnement de l'invention car cette couche compressible sert uniquement d'interface. De préférence, cette couche compressible **21** correspond à un tricot réalisé à base de polyamide et d'élasthanne. De préférence, cette couche compressible **21** est de couleur blanche. Le complexe **18** comporte une couche supérieure formée par une couche de support **20** opaque. De préférence, cette couche de support **20** est réalisée à base de polycarbonate de couleur verte.

Ce complexe **18** permet ainsi de transmettre la forme de la voute plantaire de l'utilisateur jusqu'à la surface translucide **11** afin de révéler la forme des zones d'appui par fixation de la lumière émise par la source de lumière **15**.

Un appareil de capture d'images **16**, disposé sous la surface translucide **11**, permet de transmettre la forme des zones d'appui à l'écran tactile **32**. En variante, l'appareil de capture d'images **16** peut être disposé à d'autres emplacements de la partie basse **40** et obtenir une image de la face inférieure **13** de la surface translucide **11** par réflexion sur un ou plusieurs miroirs.

A partir de l'image des zones d'appui de l'utilisateur, un algorithme implémenté dans une unité centrale reliée à l'écran tactile **32** permet de catégoriser la forme de la voute plantaire de l'utilisateur et de conseiller l'utilisateur sur des semelles, des chaussons ou des chaussures adaptées à sa morphologie.

Afin d'améliorer le guidage des produits pour l'utilisateur, le podoscope numérique **10** effectue également une mesure de la longueur et de la largeur des pieds. Cette mesure peut être effectuée simultanément ou non à la mesure des zones d'appui.

Pour effectuer cette mesure, le podoscope numérique **10** présente un second appareil de capture d'images **30** fixé sur le montant **41** et configuré pour capturer une image de la face supérieure du complexe **18**. Ainsi, lorsque l'utilisateur est positionné sur le complexe **18** en butée contre la cale **46** et avec ses pieds sensiblement droits, le second appareil de capture d'images **30** permet d'estimer la longueur et la largeur de chaque pied. Pour ce faire, la longueur et la largeur de chaque pied sur l'image issue du second appareil de capture d'images **30** est estimée en connaissant la longueur des bords du complexe **18**. L'extraction de chaque pied du complexe peut être simplifiée par l'utilisation d'un support de couleur verte ou par l'utilisation d'un support grenelé afin de limiter les réflexions des lumières externes. En outre, une source de lumière **33** peut être intégrée dans le montant **41** afin d'éliminer les ombres autour de chaque pied.

L'invention permet ainsi de mesurer les caractéristiques géométriques des pieds d'un utilisateur. L'analyse de la voute plantaire est effectuée rapidement en raison de la suppression des bruits environnants générés par les lumières externes et filtrés par la couche de support **20** opaque. En outre, le complexe **18** diminue également le risque de transmission de bactéries car l'utilisateur peut conserver ses chaussettes.

## Revendications

1. Dispositif numérique de mesure physiologique (10) comportant :
- une surface translucide (11) comportant une face supérieure (12) apte à recevoir un élément physiologique à mesurer, et une face inférieure (13), opposée à la face supérieure (12) ;
- une source lumineuse (15) en regard de la tranche de ladite surface translucide (11) de sorte qu'une lumière soit injectée dans la matière de ladite surface translucide (11) pour révéler les zones d'appui dudit élément physiologique ; et
- un appareil de capture d'images (16) configuré pour capturer une image desdites zones d'appui dudit élément physiologique ;
***caractérisé en ce que*** ledit appareil de capture d'images (16) est positionné pour observer ladite face inférieure (13) de ladite surface translucide (11), ladite face supérieure (12) étant recouverte d'un complexe (18) comportant :
- un film polymérique (19) glacé, venant au contact de ladite face supérieure (12), et
- une couche de support (20) opaque, fixée avec ledit film polymérique (19), configurée pour recevoir ledit élément physiologique.

2. Dispositif numérique de mesure physiologique selon la revendication 1, dans lequel ledit complexe (18) comporte également une couche compressible (21) disposée entre ledit film polymérique (19) et ladite couche de support (20).

3. Dispositif numérique de mesure physiologique selon la revendication 2, dans lequel ladite compressible (21) correspond à un tricot réalisé à base de polyamide et d'élasthanne.

4. Dispositif numérique de mesure physiologique selon la revendication 2 ou 3, dans lequel compressible (21) est de couleur blanche.

5. Dispositif numérique de mesure physiologique selon l'une des revendications 1 à 4, dans lequel ledit film polymérique (19) est réalisé à base de polyéthersulfone translucide, et dont l'épaisseur est comprise entre 10 et 50µm.

6. Dispositif numérique de mesure physiologique selon l'une des revendications 1 à 5, dans lequel ledit dispositif numérique (10) comporte un second appareil de capture d'images (30) positionné pour observer par-dessus ladite couche de support (20) de sorte à mesurer une longueur et/ou une largueur dudit élément physiologique.

7. Dispositif numérique de mesure physiologique selon la revendication 6, dans lequel ledit dispositif numérique (10) comporte une seconde source lumineuse (33) orientée vers ladite couche de support (20) de sorte à limiter les ombres autour dudit élément physiologique pour les mesures effectuées par ledit second appareil de capture d'images (30).

8. Dispositif numérique de mesure physiologique selon la revendication 6 ou 7, dans lequel ladite couche de support (20) comporte une surface supérieure grenelée de sorte à limiter les réflexions de la lumière sur ladite couche de support (20) pour les mesures effectuées par ledit second appareil de capture d'images (30).

9. Dispositif numérique de mesure physiologique selon l'une des revendications 6 à 8, dans lequel ladite couche de support (20) est de couleur verte.

10. Dispositif numérique de mesure physiologique selon l'une des revendications 1 à 9, dans lequel ladite couche de support (20) est réalisée à base de polycarbonate.

11. Dispositif numérique de mesure physiologique selon l'une des revendications 1 à 10, dans lequel ledit dispositif numérique (10) est un podoscope numérique.

12. Dispositif numérique de mesure physiologique selon la revendication 11, dans lequel ledit dispositif numérique (10) comporte un écran tactile (32) comportant une interface de dialogue avec un utilisateur afin de le guider lors de la prise de mesures.

13. Dispositif numérique de mesure physiologique selon la revendication 11 ou 12, dans lequel ledit dispositif numérique (10) comporte une caméra (35) apte à capturer et transmettre la position des jambes d'un utilisateur au cours du temps afin de contrôler que les jambes de l'utilisateur soient dans une position conforme à une prise de mesure.
